# FASCICULE DE BREVET EUROPEEN

(11) **EP 0 487 418 B1**
(45) Date de publication et mention de la délivrance du brevet: **22.01.1997**
(21) Numéro de dépôt: 91403133.1
(22) Date de dépôt: 20.11.1991
(51) Int. Cl.: A61L 27/00

(54) **Dispositif à usage ophtalmologique formé d'un substrat polymérique comportant des groupements fluorés en surface, et procédé d'obtention**
Ophtalmologisches Stück aus einem auf der Oberfläche Fluorgruppen enthaltenden Polymer sowie dessen Herstellungsverfahren
Ophtalmological device formed by a polymeric substrate having fluorine-containing linking groups in its surface, and its method of manufacture

(30) Priorité: 21.11.1990 FR 9014536
(43) Date de publication de la demande: 27.05.1992
(73) Titulaire: ASSOCIATION POUR LES TRANSFERTS DE TECHNOLOGIE DU MANS, 72000 Le Mans (FR); LABORATOIRES DOMILENS SOCIETE ANONYME, 69007 Lyon (FR)
(72) Inventeur: Bechetoille, Alain, F-49100 Angers (FR); Legeay, Gilbert, F-72650 la Milesse (FR); Legeais, Véronique, F-72000 Le Mans (FR); Gravagna, Philippe, F-69540 Irigny (FR); Mercier, Lionel, F-69420 Condrieu (FR)
(74) Mandataire: Guerre, Dominique

(56) Documents cités:
- EP-A- 0 096 573
- EP-A- 0 309 345
- WO-A-87/01040
- WO-A-88/08287
- FR-A- 2 446 682
- US-A- 3 740 325
- US-A- 4 655 770
- US-A- 4 656 083
- BIOMATERIALS, vol. 3, no. 2, avril 1982, pages 68-77, Butterworth & CO., (Publishers) Ltd, Guilford, Surrey, GB; H. YASUDA et al.: "Biomedical applications of plasma polymerization and plasma treatment of polymer surfaces"

## Description

La présente invention a pour objet un nouveau dispositif à usage ophtalmologique formé de substrats polymériques comportant à leur surface des groupements contenant des atomes de fluor liés chimiquement aux polymères.

Elle a aussi pour objet un procédé pour l'obtention d'un tel dispositif .

On connaît dans l'art antérieur des traitements de dispositifs à usage ophtalmologique , tels que des lentilles intraoculaires, afin d'augmenter leur biocompatibilité .

Notamment ,l'article de MATEO et RATNER (Investigative Ophtalmology and Visual Science , 3O, N°5 , 1989 ) a pour objet un traitement de lentilles intraoculaires constituées de poly(méthyl méthacrylate) (PMMA) par divers plasmas, induisant la formation d'un film à la surface de ladite lentille.Ces films sont obtenus par traitement par du perfluoropropane, de l'oxyde d'éthylène, du 2-hydroxyéthyl méthacrylate (HEMA) et du n-vinyl-2-pyrrolidone (NVP). Selon les auteurs, le traitement par le perfluoropropane induit les dommages les plus faibles sur la couche de cellules endothéliales.

Les demandes Internationales PCT WO 87 O1040 et WO 88.O8.287 ont pour objet une méthode similaire permettant de recouvrir une lentille intraoculaire par un film polymérisé à base de monomère fluorocarboné tel qu'un perfluoropropane , un perfluoropropène , un hexafluoroéthane ou un tétrafluoroéthylène .

D'autres méthodes n'utilisant pas de traitement par plasmas ont fait l'objet de dépôts de demandes de brevets . Il s'agit notamment de méthodes de traitement du PMMA par une solution d'un composé fluorocarboné (US 4 655 77O), de traitement par un polymère de silicone contenant du vinyle (US 4731080), de traitement de polyméthylméthacrylate par de l'acide hyaluronique (EP-A-233 7O8), de traitement par des solutions contenant des silanes ( Demande PCT WO 8904329 ou de traitement de poly(méthyl méthacrylate) par un film de carbone (EP-A-28O.215).

A la connaissance des inventeurs tous les traitements décrits dans l'art antérieur présentent des inconvénients notables en ce qui concerne la biocompatibilité, ou les propriétés mécaniques des substrats traités.

Ces inconvénients , inhérents aux traitements utilisés sont principalement :
- la formation d'une couche à la surface de lentilles entraîne une augmentation de leurs épaisseurs qui altère leurs caractéristiques mécaniques et optiques. L'épaisseur peut d'autre part être augmentée de manière hétérogène.
- un manque d'adhérence entraînant des départs de matière ,
- la couche formée peut présenter, outre une faible biocompatibilité, une friabilité importante réduisant encore sa biocompatibilité ,
- l'immersion dans des solutions de traitement entraîne une contamination des surfaces des lentilles par des réactifs fixés de manière non covalente, ce qui requiert une étape de lavage.Les produits lavés à cette étape ne remplissent pas nécessairement les normes en vigueur en ophtalmologie, en ce qui concerne la présence d'impuretés.

D'autre part, une méthode de traitement d'articles en matériaux polymériques pour présenter une hémocompatibilité améliorée et une thrombogénéicité diminuée a fait l'objet du dépôt de la demande FR 87 13 154. Cette méthode comprend le traitement d'articles constitués de matériaux polymériques contenant des atomes d'hydrogène par un plasma de molécules fluorées non polymérisables, dans des conditions ménagées . Ce traitement conduit au remplacement d'atomes d'hydrogène en surface par des atomes de fluor, ou des groupements CF, CF₂ ou CF₃ , le fluor total représentant au plus lO% des atomes présents en surface . Les matériaux pouvant être traités par cette méthode sont notamment le polyéthylène, le polychlorure de vinyle, ou le polyéthylène téréphtalate . Le plasma utilisé de manière préférentielle est composé de molécules de tétrafluorométhane.

De manière avantageuse le traitement est effectué pendant des durées allant de quelques secondes à une dizaine de minutes à une puissance d'émission allant de O,l watt à 2 watts par litre de capacité de réacteur.

Les articles traités sont en particulier des cathéters, des seringues ou des prothèses vasculaires ou cardiaques.

Il est donc clair que cette méthode de traitement concerne des dispositifs en contact spécifiquement avec le sang , et non avec des liquides ou des tissus de l'oeil. Les matériaux et leurs conditions de traitement sont liés aux impératifs du contact avec le sang.

De même , le brevet US-A-3.74O.325 mentionne la substitution d'atomes d'hydrogène par des atomes de fluor ou des groupements fluorés afin de rendre la surface d'un matériau plus résistante à la corrosion et aux souillures ou plus inerte chimiquement que celle du matériau non traité .

Aucune application spécifique à des matériaux en contact avec des liquides ou des tissus de l'oeil n'est mentionnée .

Les inventeurs ont montré de manière surprenante que l'on pouvait traiter des dispositifs à usage ophtalmologique par des plasmas de molécules fluorées , afin d'augmenter leur biocompatibilité tout en évitant la formation de couches supplémentaires comme dans les méthodes décrites dans l'art antérieur.

La présente invention a donc pour objet un dispositif à usage ophtalmologique , notamment implant ou lentille intraoculaire comprenant un substrat polymérique, ledit substrat contenant des atomes d'hydrogène et ayant des qualités mécaniques traditionnellement requises pour ce type de dispositif, caractérisé par le fait que les atomes d'hydrogène présents à la surface dudit dispositif ont été remplacés par des atomes de fluor, ou des groupements CF, CF₂ ou CF₃ , le fluor total représentant au moins l5% des atomes présents sur cette surface , avantageusement au moins 3O% et de préférence de 3O à 5O% des atomes présents sur cette surface .

La surface du dispositif est définie comme étant la couche superficielle d'environ l5O angströms d'épaisseur.

De manière préférentielle, ledit substrat polymérique possède des qualités optiques et est notamment le poly(méthyl méthacrylate) (PMMA), le 2-hydroxy éthyl-méthacrylate (HEMA), le silicone , ou un polysulfonate.

Ces dispositifs présentent des biocompatibilités améliorées par rapport à ceux décrits dans l'art antérieur, et du fait de l'absence de couches supplémentaires présentent en outre une épaisseur identique et une absence d'effritement en surface. Ces matériaux ont conservé des qualités optiques permettant la transmission des rayons lumineux dans une gamme de longueurs d'onde visible .

De tels dispositifs, outre les implants intraoculaires tels que des lentilles ou les implants pour glaucomes, peuvent aussi comprendre des fils pour suture.

La présente invention a d'autre part pour objet un procédé d'obtention des dispositifs tels que décrits précédemment comprenant le traitement de surface du dispositif se trouvant dans sa forme appropriée pour son utilisation, par un plasma de molécules fluorées non polymérisables, à une puissance d'émission du réacteur comprise entre 3 et 10 watts environ par litre de capacité de réacteur.

Avantageusement, le temps de traitement de la surface est compris entre l et 20 minutes environ.

Selon un mode de mise en oeuvre particulier de l'invention, la puissance d'émission du réacteur est d'environ 7 watts par litre et le temps de traitement d'environ lO minutes.

Le traitement peut être effectué sous vide, préférentiellement à une pression comprise entre O,l et l Torr.

Les molécules fluorées peuvent être toutes molécules non-polymérisables adaptées à ce type de procédé , et en particulier le CF₄ et le SF₆ qui sont facilement disponibles sous forme de gaz.

Ce procédé de traitement de surface permet la stérilisation des dispositifs après le traitement . Cette caractéristique , constitue un avantage non négligeable dans la pratique dudit procédé.

Le procédé selon l'invention possède aussi l'avantage de ne pas nécessiter de températures de traitement élevées, les températures auxquelles se font ces traitements dans les plasmas étant comprises entre 2O°C et 8O°C . Cette gamme de températures permet de traiter ces dispositifs sans échauffement notable , donc sans risque d'altération dimensionnelle.

Le traitement en surface par substitution des atomes d'hydrogène , c'est-à-dire sans formation d'une couche supplémentaire , possède l'avantage outre d'éviter des risques de délaminage entre la couche et le reste du dispositif , de ne pas modifier la géométrie , ni les dimensions et de ne pas entraîner d'hétérogénéité morphologique.

De plus,le procédé objet de la présente demande présente une bonne reproductibilité et assure la non-introduction d'impuretés à la surface du dispositif traité .

Cette reproductibilité se retrouve tant au niveau qualitatif (nature des groupements greffés) que quantitatif (concentration relative des différents groupements). Il est aussi envisageable d'introduire un contrôle de routine pour confirmer la fiabilité du traitement , par exemple par mesure du mouillage ou par analyse des signaux du carbone et du fluor ce qui permet ainsi une vérification rapide de la qualité du traitement.

La description qui suit donne à titre illustratif et non limitatif des exemples d'application de l'invention.

Les figures l à l7 sont des photographies obtenues en microscopie électronique à balayage de lentilles traitées par le procédé selon l'invention et mises en contact avec des leucocytes.

### EXEMPLE 1 - Procédé de traitement d'implants intraoculaires.

Les implants utilisés dans cet exemple sont des lentilles de divers types , soit à palet non usiné et non poli manuellement (échantillons A à C) , soit à palet usiné de plan convexe (rayon de courbure correspondant à une optique biconvexe de 23 dioptries) et poli manuellement(échantillon D à F).

Un témoin "plaque" correspond au fond du récipient contenant les échantillons lors du traitement a aussi été traité .

Le traitement est effectué dans des plasmas à basse température avec des gaz fluorés, en l'occurrence CF₄, durant 5 ou lO minutes dans un réacteur de 15 litres émettant à 13,56 MHz. Les puissances appliquées sont de 5O ou lOO watts suivant les échantillons .

Chaque essai a été réalisé trois fois.

Les propriétés des lentilles ainsi traitées ont été analysées, par analyse chimique par spectroscopie des électrons (ESCA), et en ce qui concerne leur biocompatibilité, comme indiqué dans les exemples 2 et 3.

### EXEMPLE 2 - Analyse ESCA des lentilles obtenues dans l'exemple l.

Les lentilles traitées dans l'exemple l , ont été analysées par la technique ESCA (Analyse Chimique par Spectroscopie des Electrons). Les résultats obtenus sont résumés dans les tableaux l à 4.

Les tableaux l et 2 correspondent aux contenus en carbone , oxygène, fluor et azote tels que déterminés par analyse ESCA respectivement des palets non usinés et usinés.

Les tableaux 3 et 4 représentent les quantités des diverses fonctions chimiques à la surface respectivement des palets usinés et non usinés tels que déterminés par la méthode ESCA.

Les lentilles A et D ont été traitées en 5 minutes à 5O watts, les lentilles B et E ont été traitées lO minutes à 5O watts et les lentilles C et F ont été traitées 5 minutes à lOO watts.

Les résultats obtenus montrent que :

Tous les échantillons analysés ont subi une modification importante de leur surface : greffage de fluor et apparition de nouvelles fonctions carbonées (-CF; -CF₂; CF₃).

On constate sur tous les échantillons une présence d'azote, cet élément est probablement apporté par le traitement par le plasma, et peut être dû à un mauvais vide.

En ce qui concerne l'analyse des surfaces traitées:
- le traitement des séries A et D conduit au taux de fluoration le plus faible;
- les deux autres traitements (séries B, E et C,F) conduisent à une fluoration nettement plus importante (F/C maximum pour le troisième traitement, c'est-à-dire les séries C et F).

Toutefois, on observe une nette distinction entre la série des palets usinés et non usinés. Le taux de fluoration est, dans tous les cas, beaucoup plus élevé pour les palets non usinés .

On note une homogénéité des résultats pour les trois échantillons de chaque série dans le cas des palets non usinés. Les compositions élémentaires sont homogènes d'un échantillon à l'autre; il en est de même pour la répartition des différentes fonctions carbonées.

Les échantillons des séries B, E et C, F présentent des surfaces où l'on retrouve en plus grande concentration les fonctions CF₂ et CF₃ .

Les résultats analysés ci-dessus montrent que malgré une homogénéité imparfaite dans certains cas , on obtient des lentilles présentant un taux de fluoration important.

### EXEMPLE 3- Analyse de la biocompatibilité des lentilles traitées selon l'exemple l.

### 1) Protocole expérimental:

### a) Principe.

Du sang humain est prélevé chez un donneur sain.

Les leucocytes sont obtenus par sédimentation dans du Dextran puis dans un gradient de Percoll.

Par donneur, trois lentilles de chaque type sont testées par contact dans des puits d'une microplaque avec une suspension de lO⁵ cellules et une solution de ferricytochrome C .

La génération de l'anion superoxyde est déterminée par mesure spectrophotométrique de la réduction du ferricytochrome C à 55O nm en continu pendant 2 heures à 37°C sur un lecteur Vmax Molecular Devices et enregistrée sur un microordinateur IMB PS/2 sous logiciel Soft-Max.

L'étude est réalisée sur du sang humain provenant de trois donneurs différents.

En fin d'expérimentation une lentille de chaque type est analysée en microscopie électronique à balayage.

### b) Méthode:

Par donneur, 5O ml de sang sont prélevés dans un flacon en verre contenant une solution anticoagulante (CDP,Centre de Transfusion Sanguine de Lyon , France ) de composition suivante :

| | |
|---|---|
| acide citrique lH₂O | O,32g |
| citrate trisodique 2H₂O | 2,58g |
| phosphate monosodique 2H₂O | O,247g |
| glucose lH₂O | 2,55g |
| eau distillée qsp | lOO ml |

Le sang fraîchement prélevé est mis en contact dans des tubes stériles avec du Dextran (Dextran T5O, Pharmacia-LKB Biotechnology AB ), pendant environ une heure à température ambiante .

Le surnageant est décanté et centrifugé.

Les cellules présentes dans le culot de centrifugation sont lavées deux fois dans une solution de PBS (PBS Dulbeco's, GIBCO) puis traitées par un gradient de Percoll (Pharmacia) afin d'obtenir les leucocytes totaux.

Ces leucocytes sont lavés deux fois dans une solution de PBS puis numérés et ajustés dans une solution HEPES (Laboratoires Flow) à la concentration de 1 million de cellules par millilitre.

Dans une microplaque 96 puits (Falcon 3872 Primaria) sont déposées stérilement trois lentilles de chaque type à étudier ,la face à évaluer n'étant pas en contact avec le fond de la cupule.

L'ordre dans lequel sont déposées les lentilles est tiré au sort.

Dans chaque puits contenant une lentille sont introduits lOO µl d'une suspension leucocytaire à 1 million de cellules par millilitre et lOO µl d'une solution de ferricytochrome C(SIGMA).

L'étude est réalisée sur du sang provenant de trois donneurs différents.

Pour chaque série un "témoin plaque" (sans lentille) est réalisé.

Les évolutions des variations de densité optique correspondant à la réduction du ferricytochrome par l'anion superoxyde sont mesurées à 37°C , à la longueur d'onde de 55O nm pendant deux heures toutes les minutes, sur un lecteur de plaques V MAX Molecular Devices et enregistrées sur un microordinateur IBM PS/2 sous logiciel Soft-Max.

Les cinétiques de génération de l'anion superoxyde sont évaluées par le paramètre p, pente maximale de la courbe (calculée sur 25 points) correspondant à la vitesse de production de l'anion superoxyde.

En fin d'expérimentation, après un contact de deux heures avec les leucocytes , un échantillon de chaque type de lentille est prélevé, fixé dans une solution de glutaraldéhyde cacodylate et analysé en microscopie électronique à balayage.

### 2) Résultats obtenus.

### a) Analyse des cinétiques de génération de l'anion superoxyde .

Les valeurs des pentes maximales de cinétique de génération de l'anion superoxyde sont consignées sur les tableaux 5, 6 et 7 correspondant aux leucocytes de 3 sujets (n°1, 2 et 3).

Dans les conditions de l'expérimentation, les valeurs moyennes (n=9) des pentes maximales (mDO/mn) des cinétiques des réactions lors de l'évaluation des produits sont :

| | |
|---|---|
| A* | O,71 ± O,27 |
| B | O,88 ± O,41 |
| C | O,82 ± O,32 |
| D | 0,92 ± 0,29 |
| E | 0,88 ± 0,35 |
| F | 0,86 ± 0,25 |
| G | 2 ± 0,40 |
| Témoin plaque (T). | l,l2 ± 0,37 |

| | |
|---|---|
| * (n=8) | |

### b)Analyse des échantillons en microscopie électronique à balavage.

### Echantillon A

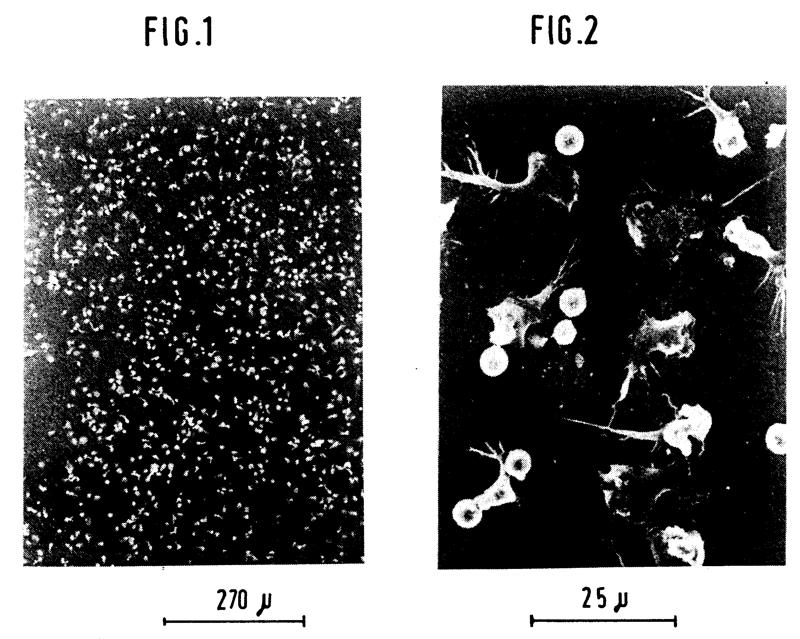

La surface de la lentille est recouverte à 7O% par des éléments cellulaires ( fig.1 ) généralement adhérents et dont certains montrent des figures d'excrétion (fig.2).

Les cellules les moins adhérentes semblent être des lymphocytes .

### Echantillon B

Peu d'éléments cellulaires ont adhéré à la surface de cette lentille. On ne remarque la présence que de trois petits foyers sur la totalité de la lentille, localisés vers les bords à l'intérieur desquels les cellules sont adhérentes, certaines ayant excrété .

### Echantillon C.

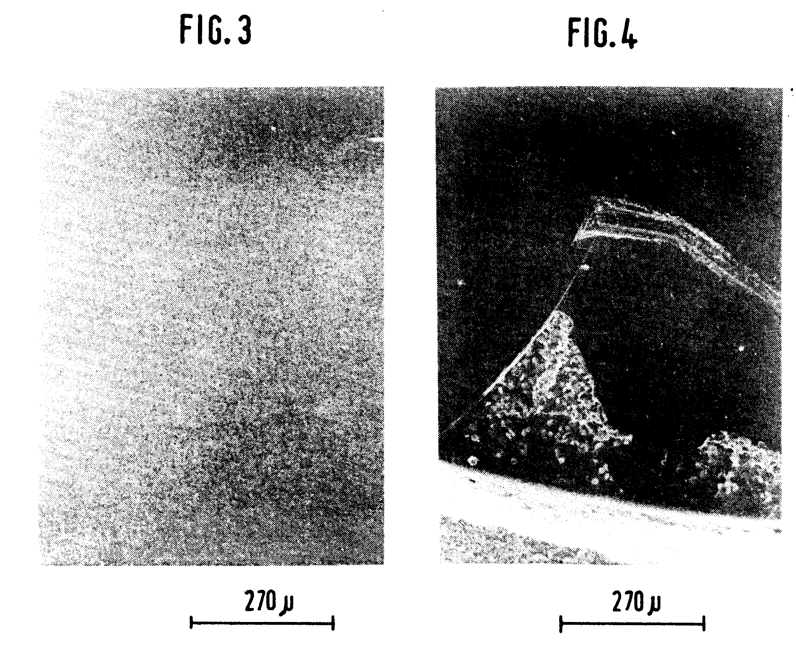

La surface n'est recouverte d'aucun élément cellulaire , elle est libre de toute adhésion et excrétion cellulaire (fig.3) . A noter un état de surface dépourvu de tout accident et uniformément lisse.

### Echantillon D

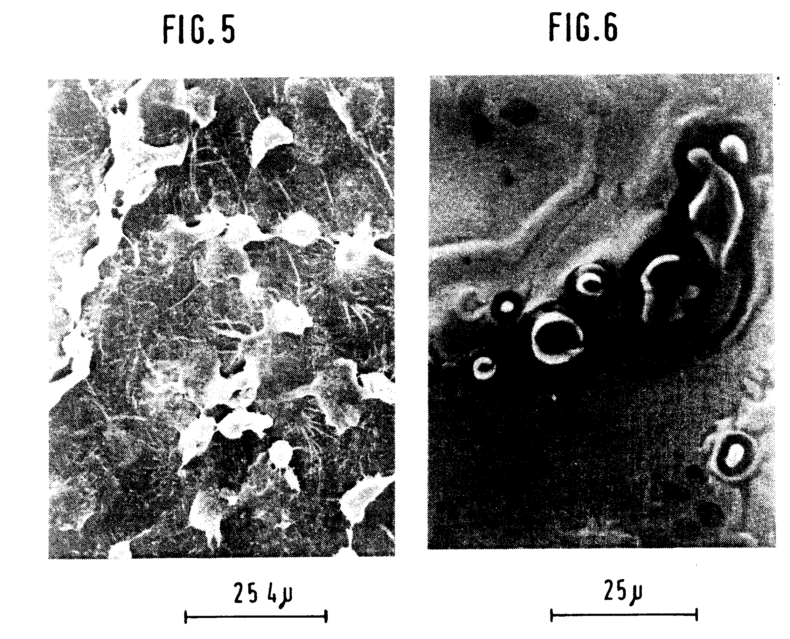

La surface de la lentille n'est recouverte que par 2 petits foyers cellulaires , localisés vers les bords (fig.4) . Le reste de la lentille est dépourvu d'éléments cellulaires, mais l'état de surface présente des imperfections sous forme de dépôts plus ou moins organisés en figures géométriques (fig.5) , de même qu'un soulèvement partiel de la couche superficielle avec une émergence d'éléments arrondis (fig.6).

### Echantillon E.

Aucun élément cellulaire n'est présent à la surface de la lentille, mais on remarque les mêmes imperfections de surface que celles observées sur l'échantillon précédent, à savoir :
- des dépôts plus ou moins géométriques
- un soulèvement partiel de la surface avec émergence d'éléments arrondis .

Sur cet échantillon le décollement de la couche superficielle se traduit également par des figures "étoilées".

### Echantillon F

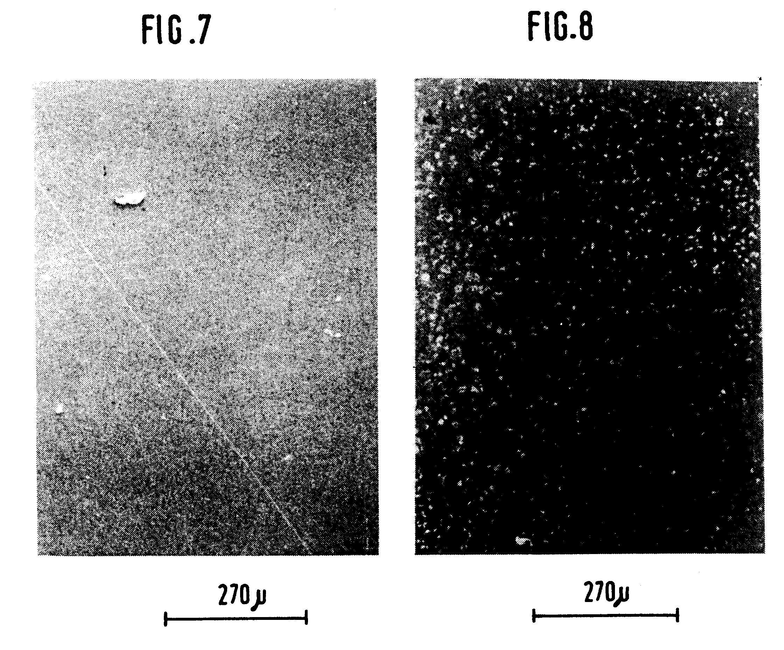

La lentille n'est pas recouverte, elle est libre de toute adhésion et excrétion cellulaire (fig.7). La surface est lisse excepté la présence d'une rayure qui est certainement de nature artéfactuelle suite au prélèvement de l'échantillon.

### Echantillon G (témoin non traité).

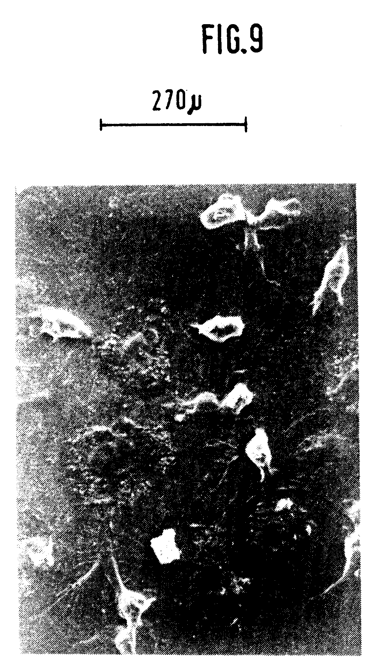

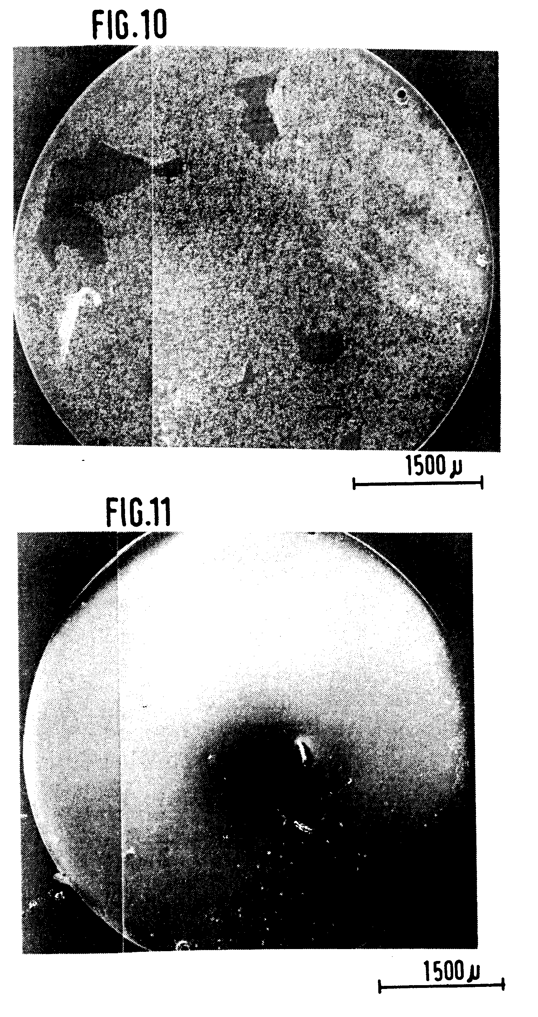

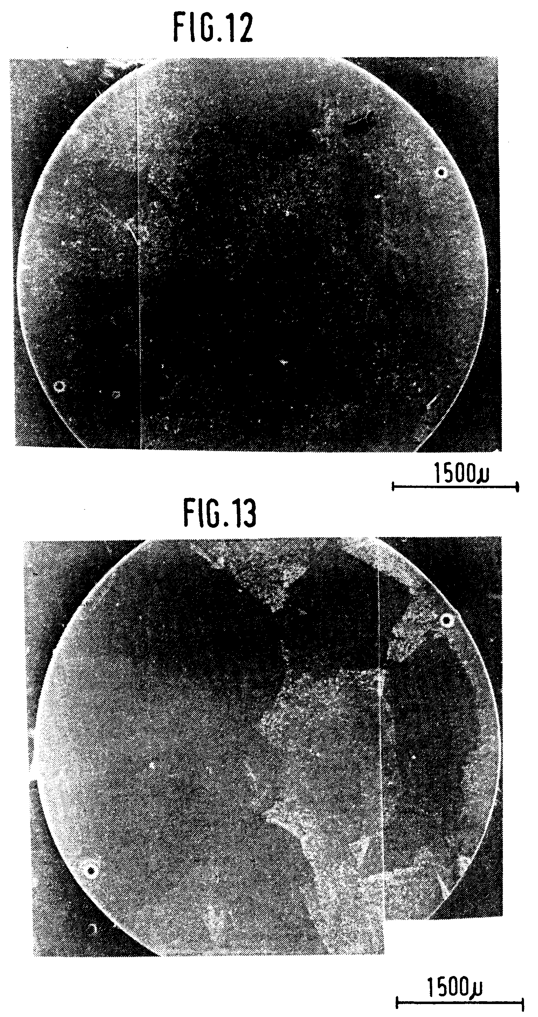

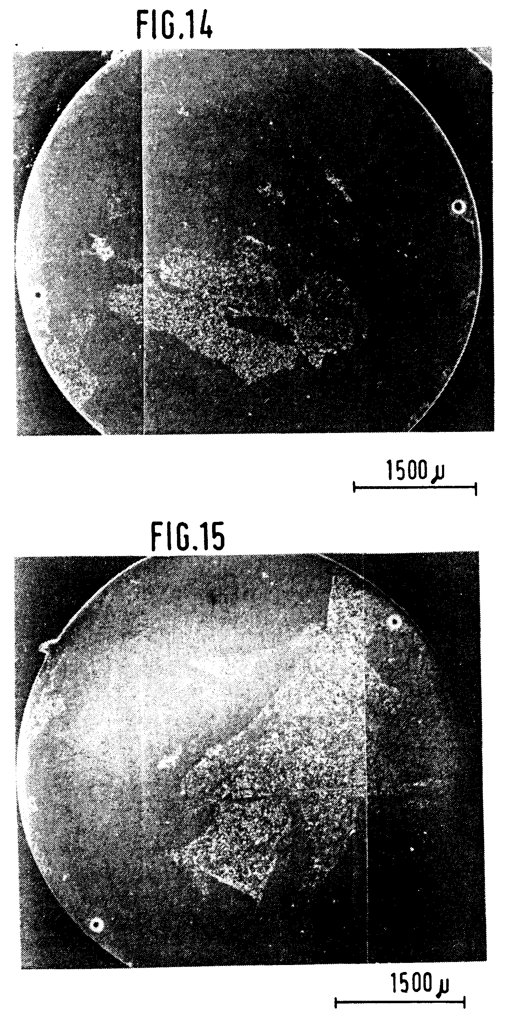

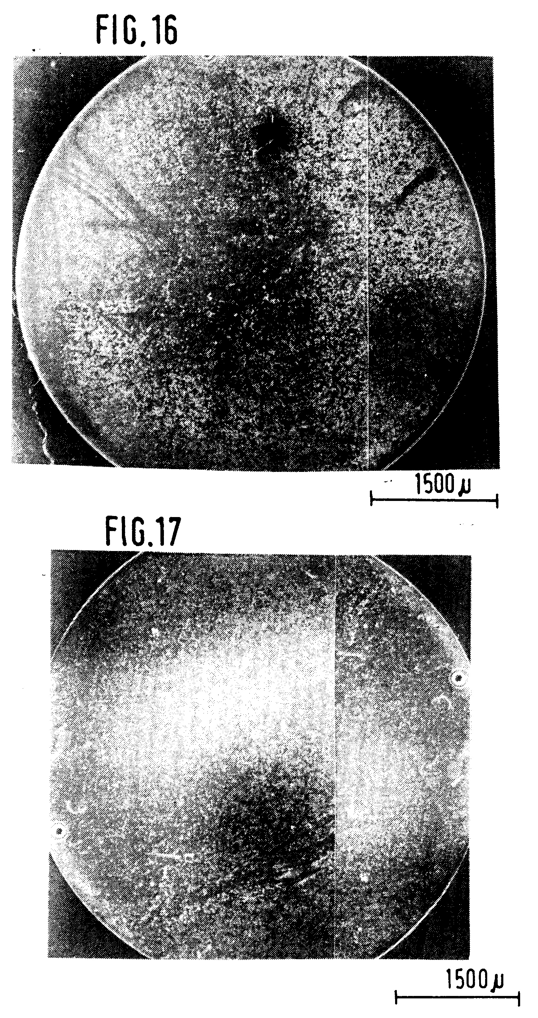

La surface de la lentille est totalement recouverte par des éléments cellulaires et des protéines (Fig.8) . La majorité des cellules sont très adhérentes et un grand nombre d'entre elles ont excrété ; le produit de leur excrétion tapisse toute la surface de l'échantillon (fig.9).

### c) Conclusion:

L'activation de leucocytes humains au contact de différentes surfaces intraoculaires a été étudiée:
- en suivant l'apparition d'un marqueur, l'anion superoxyde,
- en analysant la surface après 2 heures de contact en microscopie électronique à balayage.

La génération de l'anion superoxyde exprimée par la pente maximale de la courbe est augmentée dans les puits en présence des lentilles de type G c'est-à-dire non traitées , par rapport aux puits ne contenant pas de lentille (puits T) . A l'opposé les lentilles de type A, B, C, D, E et F ne modifient pas la génération de l'anion superoxyde par rapport au puits témoin (puits T).

Les traitements subis par les lentilles A, B, C, D, E et F, ont diminué la réactivité de leur surface vis-à-vis des leucocytes humains. Il n'est pas possible d'identifier au sein du groupe des surfaces traitées, un traitement spécifique.

L'examen en microscopie à balayage (réalisé sur une expérience), rend compte d'un état des surfaces après 2 heures d'incubation au contact de leucocytes . Alors que la lentille non traitée (G) est le siège d'une importante adhésion/activation cellulaire , sur un film protéique complet, les surfaces traitées sont généralement, sauf pour la surface A, moins recouvertes de protéines et d'éléments cellulaires.

Les surfaces C, E et F sont exemptes de toute adhésion cellulaire.

### EXEMPLE 4 - Activation des cellules inflammatoires

L'objet de l'étude est d'évaluer en microscopie électronique à balayage , par des critères quantitatifs ou semi-quantitatifs, l'importance de l'activation des cellules inflammatoires ( leucocytes humains) par différentes surfaces de lentilles intraoculaires et après différents temps de contact .

Quatre types de lentilles ont été testés dans ces essais :
lentille A traitée par le CF₄ à 5O watts durant lO minutes ;
lentille B traitée par le CF₄ à lOO watts durant 5 minutes ;
lentille C traitée par le CF₄ à lOO watts durant lO minutes;
lentille D : lentille témoin non traitée.

Ces quatre types de lentilles présentent un plan convexe .

### l°) Protocole expérimental :

Par donneur, 5O ml de sang sont prélevés dans un flacon en verre contenant une solution anticoagulante (CPD).

Le sang fraîchement prélevé est mis en contact dans des tubes stériles avec du Dextran, pendant environ une heure à la température ambiante .

Le surnageant est décanté et centrifugé.

Les cellules présentes dans le culot de centrifugation sont lavées deux fois dans une solution de PBS , puis traitées par un gradient de Percoll afin d'obtenir les leucocytes totaux .

Ces leucocytes sont lavés deux fois dans une solution de PBS puis numérés et ajustés dans une solution HEPES/HBSS à la concentration de 1 million de cellules par millilitre.

Dans une microplaque 96 puits on dépose stérilement une lentille de chaque type à étudier (la face à évaluer n'étant pas en contact avec le fond de la cupule).

Dans chaque puits contenant une lentille sont introduits lOO µl d'une suspension leucocytaire à l million de cellules par millilitre.

Après un temps de contact de 3O minutes, l heure, 2 heures et 4 heures à 37°C, les échantillons sont prélevés et fixés dans un mélange de glutaraldéhyde 4% tamponné au cacodylate de sodium O,2 M (v/v) pendant l jour.

Ils sont ensuite rincés plusieurs fois au cacodylate de sodium O,2 M , puis subissent une déshydratation éthylique progressive suivie d'une déshydratation au Fréon.

Les échantillons sont alors métallisés à l'Or-Palladium, puis observés au microscope électronique à balayage (HITACHI S 8OO) à une tension d'accélération des électrons de lO KV.

Les solutions utilisées ont été répertoriées dans l'exemple 3.

Le mode de prélèvement des lentilles en fin d'expérimentation a différé au cours des 3 essais .

Dans l'essai l, les lentilles ont été prélevées par la face en contact avec les cellules , ce qui a pu occasionner des déchirements ou des décollements du tapis cellulaire, alors que pour les essais 2 et 3 elles ont été prélevées par la face non en contact.

### 2°) RESULTATS

### ESSAI l . (voir tableau 8 ci-après).

### Lentille A

Elle est très rapidement activatrice; à 3O minutes (Fig.lO) elle est totalement recouverte de cellules très adhérentes dont certaines sont en phase d'excrétion et des débris cellulaires sont déjà visibles.

En revanche cette activation ne se poursuit pas au cours du temps ; à l heure (figure ll) le tapis cellulaire a presque disparu.

### Lentilles B et C.

Elles sont moins activatrices et tout particulièrement la lentille C.

Le recouvrement cellulaire est moindre et il existe dans les temps courts de nombreuses cellules ne développant qu'une faible interaction avec les lentilles.

Les figures l2 et l3 sont relatives au recouvrement de B à 3O minutes et l heure .

Les figures l4 et l5 concernent la lentille C (3O minutes et l heure ).

### LENTILLE D

Elle présente la surface la plus fortement activatrice vis-à-vis des cellules leucocytaires; le recouvrement de la lentille est presque total durant 2 heures au moins, les cellules présentes sont très adhérentes et de nombreuses cellules sont en phase d'excrétion.

A 4 heures le tapis cellulaire a complètement disparu.

Les figures l6 et l7 représentent le recouvrement à 3O′ et à l heure.

### ESSAI 2 (voir tableau 9 ci-après).

### LENTILLE A

Elle est très rapidement activatrice et elle le reste au moins jusqu'à une heure de contact; au-delà le tapis cellulaire a presque totalement disparu.

### LENTILLES B et C

Elles sont peu activatrices durant les 4 heures de contact ( faible recouvrement et il n'existe que peu de cellules très adhérentes et/ou ayant excrété).

### LENTILLE D

Elle est fortement activatrice et ceci durant les 4 heures de contact (le tapis cellulaire persiste à 4 heures ). Les cellules sont toutes très adhérentes, beaucoup ont excrété, il existe de nombreux débris cellulaires.

### ESSAI 3 (voir tableau ll ci-après).

Par rapport aux deux essais précédents, l'activation cellulaire est plus marquée pour chaque type de lentilles (les pourcentages de recouvrement sont plus importants et les temps d'activation plus longs ).

### LENTILLE A

Elle est fortement activatrice et la diminution du recouvrement cellulaire ne se produit qu'après 2 heures . Néanmoins il persiste une activation cellulaire à la 4ème heure .

### LENTILLES B et C.

Elles ne sont pas activatrices pendant les temps courts mais le deviennent après un temps de contact prolongé (2 heures pour la lentille B et l heure pour la lentille C).

### LENTILLE D

Elle est toujours fortement activatrice ceci durant les 4 heures de contact et dès les 3O premières minutes.

### 3°) Conclusion :

La mise en contact de 4 types de lentilles ayant reçu des traitements de surface différents, avec une lignée leucocytaire de trois sangs humains différents a permis d'établir une classification de ces matériaux en terme d'activation cellulaire . Cette classification a été obtenue au terme d'une expérimentation et d'examens réalisés en "aveugle ".

La lentille A est très activatrice mais diffère de la lentille D par une durée d'activation moins longue qui disparaît en général après l heure de contact . Les cellules en phase d'excrétion sont également un peu moins nombreuses.

Les lentilles B et C sont difficiles à différencier car elles sont toutes deux faiblement activatrices (ou activatrices après un temps de contact assez long : essai 3) . Leur recouvrement cellulaire est faible et sur ces lentilles les cellules sont généralement peu adhérentes.

La lentille D est la plus activatrice avec une activation qui persiste en général pendant les 4 heures de contact . Elle se caractérise par une surface totalement recouverte de cellules en majorité adhérentes et ayant excrété.

Les temps d'expérimentation les plus pertinents sont, compte tenu des conditions expérimentales, les durées de contact de 3O minutes et 6O minutes . Au-delà de 2 heures d'essai, il faut en effet intégrer la survie des leucocytes après les techniques d'isolement et d'expérimentation in vitro.

### EXEMPLE 5

### - Mesure de la mouillabilité.

Les quatre types de lentilles décrits à l'exemple 4 ont été testés.

La mouillabilité a été estimée par mesure de l'angle formé entre une goutte d'eau et une surface plane des lentilles .

Les résultats obtenus sont les suivants:

| | | |
|---|---|---|
| lentille A | test l | lOO° |
| | test 2 | 98° |
| | test 3 | lOO° |
| lentille B | test l | lO6° |
| | test 2 | lOO° |
| | test 3 | lO4° |
| lentille C | test l | lO3° |
| | test 2 | lOl° |
| | test 3 | lO2° |
| lentille D | | 7O-8O°. |

On ne constate pas de différence notable entre les trois conditions de traitement par le plasma (A, B et C). Par contre, on observe une meilleure mouillabilité de ces trois types de lentilles par rapport aux lentilles non traitées (D).

**TABLEAU 1**

| ANALYSE ESCA DES PALETS NON USINES | | | | | |
|---|---|---|---|---|---|
| Echantillon | | Elément en % | | | |
| | C | O | F | N | |
| 1 | 47,9 | 18,4 | 32,7 | 1,0 | Série A |
| 2 | 44,5 | 17,3 | 37,2 | 1,0 | |
| 3 | 45,6 | 19,2 | 34,1 | 1,1 | |
| 1 | 38,4 | 10,2 | 51,0 | 0,4 | Série B |
| 2 | 40,1 | 11,0 | 48,2 | 0,7 | |
| 3 | 38,4 | 10,0 | 51,6 | traces | |
| 1 | 36,8 | 8,9 | 54,3 | traces | Série C |
| 2 | 36,8 | 9,0 | 53,9 | 0,3 | |
| 3 | 45,8 | 14,7 | 38,2 | 1,3 | |

**TABLEAU 2**

| ANALYSE ESCA DES PALETS USINES | | | | | | |
|---|---|---|---|---|---|---|
| Echantillon | | Elément(en %) | | | | |
| | C | O | F | N | Al | |
| 1 | 48,8 | 16,7 | 33,1 | 1,4 | traces | Série D |
| 2 | 45,3 | 14,6 | 39,0 | 1,1 | traces | |
| 3 | 58,3 | 22,2 | 16,5 | 1,5 | 1,4 | |
| 1 | 45,7 | 17,9 | 35,6 | 0,9 | traces | Série E |
| 2 | 53,1 | 20,0 | 25,8 | 1,1 | traces | |
| 3 | 45,3 | 17,8 | 35,9 | 1,0 | - | |
| 1 | 48,7 | 18,6 | 30,6 | 2,1 | traces | Série F |
| 2 | 45,0 | 16,1 | 37,6 | 1,3 | traces | |
| 3 | 37,9 | 9,0 | 51,7 | 0,8 | 0,6 | |
| PMMA(théo) | 71,43 | 28,57 | - | - | - | |
| PMMA(ESCA) | 72,5 | 27,5 | - | - | - | |

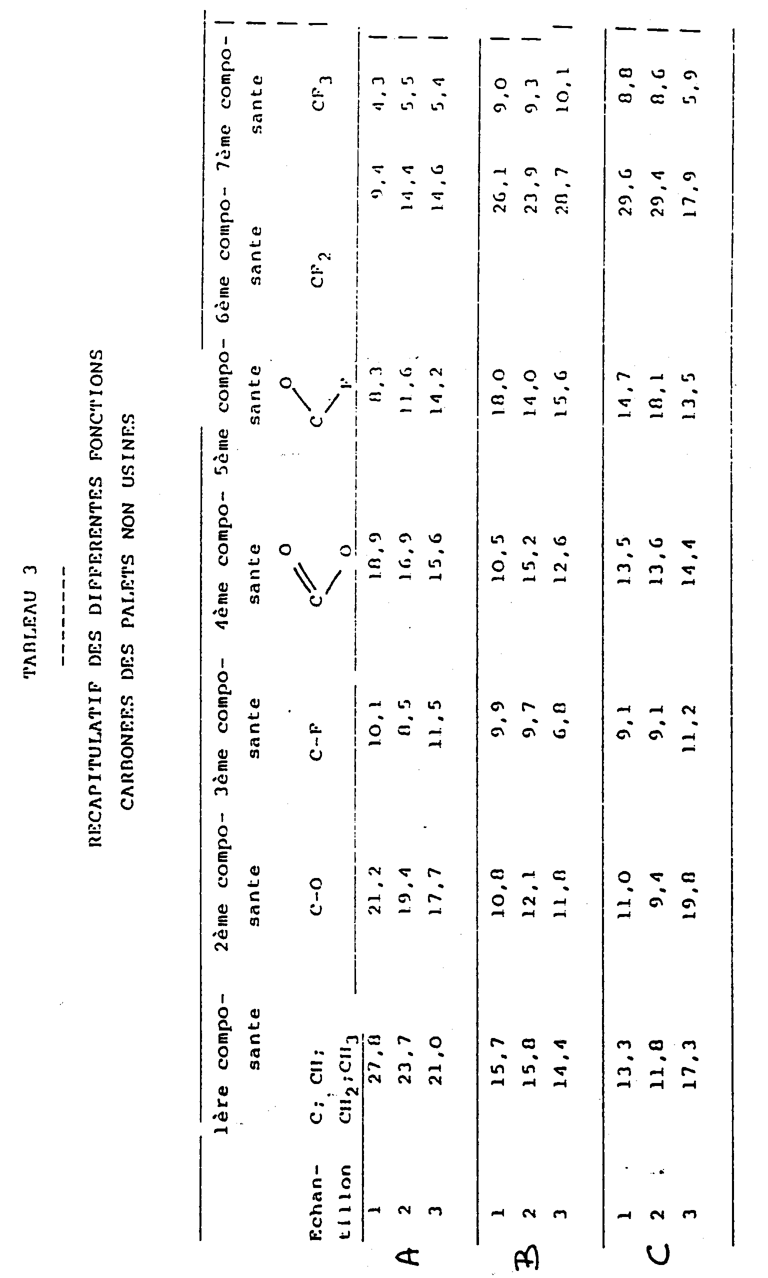

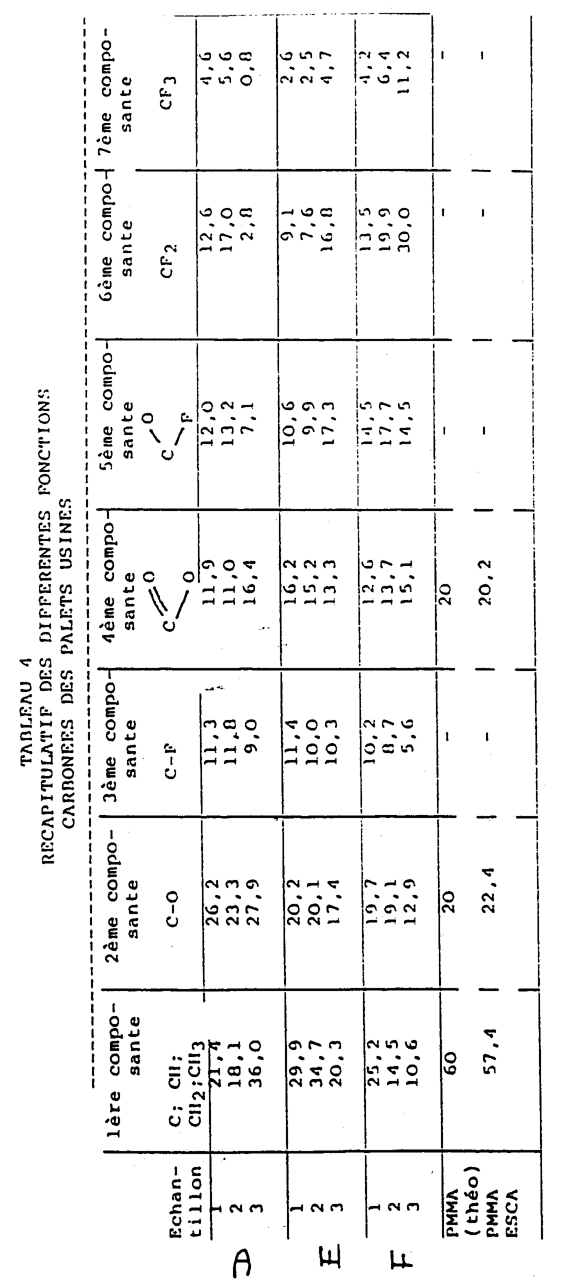

**TABLEAU 5**

| Tableau des valeurs individuelles des pentes maximales (mDO/mn) des cinétiques de chaque réaction lors de l'évaluation des produits à étudier avec les leucocytes du sujet n°1 | | | | | | | | |
|---|---|---|---|---|---|---|---|---|
| ESSAI | PRODUITS ETUDIES | | | | | | | |
| | A | B | C | D | E | F | G | T |
| n°1 | 0,391 | 0,491 | 0,565 | 0,544 | 0,457 | 0,757 | 2,039 | 0,922 |
| n°2 | 0,485 | 0,494 | 0,500 | 0,764 | 0,562 | 0,454 | 2,190 | 0,919 |
| n°3 | 0,475 | 0,465 | 0,570 | 0,675 | 0,409 | 0,585 | 1,788 | 0,825 |
| Moyenne | 0,450 | 0,483 | 0,545 | 0,661 | 0,476 | 0,599 | 2,010 | 0,889 |

**TABLEAU 6**

| Tableau des valeurs individuelles des pentes maximales (mDO/mn) des cinétiques de chaque réaction lors de l'évaluation des produits à étudier avec les leucocytes du sujet n°2 | | | | | | | | |
|---|---|---|---|---|---|---|---|---|
| ESSAI | PRODUITS ETUDIES | | | | | | | |
| | A | B | C | D | E | F | G | T |
| n°1 | 0,818 | 1,541 | 0,734 | 1,117 | 0,994 | 0,912 | 1,555 | 0,722 |
| n°2 | 1,221 | 1,062 | 0,682 | 1,159 | 1,014 | 0,755 | 1,802 | 0,892 |
| n°3 | 0,809 | 1,278 | 0,739 | 1,046 | 0,780 | 0,853 | 1,678 | 1,142 |
| Moyenne | 0,949 | 1,249 | 0,718 | 1,107 | 0,929 | 0,840 | 1,678 | 0,919 |

**TABLEAU 7**

| Tableau des valeurs individuelles des pentes maximales (mDO/mn) des cinétiques de chaque réaction lors de l'évaluation des produits à étudier avec les leucocytes du sujet n°3 | | | | | | | | |
|---|---|---|---|---|---|---|---|---|
| ESSAI | PRODUITS ETUDIES | | | | | | | |
| | A | B | C | D | E | F | G | T |
| n°1 | - | 0,584 | 0,981 | 0,823 | 1,172 | 1,138 | 1,946 | 1,278 |
| n°2 | 0,791 | 1,251 | 1,484 | 0,686 | 1,389 | 1,153 | 2,054 | 1,744 |
| n°3 | 0,691 | 0,768 | 1,096 | 1,446 | 1,137 | 1,158 | 2,933 | 1,651 |
| Moyenne | 0,741 | 0,868 | 1,187 | 0,985 | 1,233 | 1,150 | 2,311 | 1,158 |

**TABLEAU 8**

| ESSAI 1 | | POURCENTAGE DE RECOUVREMENT | CELLULES DE LA LIGNEE INFLAMMATOIRE | | | DEURIS CELLULAIRES | FILM PROTEIQUE |
|---|---|---|---|---|---|---|---|
| | | | très faiblement adhérentes | ayant adhéré | ayant excrété | | |
| A | 30 | 90% | + | +++ | ++ | + | |
| | 1H | < 5% | | + | + | + | |
| | 2H | 8 à 10% | | + | + | + | |
| | 4H | < 5% | | + | + | + | |
| B | 30 | 75% | +++ | ++ | + | + | |
| | 1 H | 35% | + | ++ | ++ | ++ | |
| | 2 H | < 5 % | | + | + | + | |
| | 4 H | 55% | | +++ | + | + | |
| C | 30 | 20% | +++ | + | + | + | + |
| | 1 H | 30% | ++ | ++ | ++ | + | + |
| | 2 H | < 5% | | + | + | + | + |
| | 4 H | < 5% | | + | + | + | |
| D | 30 | 98% | + | +++ | +++ | ++ | |
| | 1H | 100% | + | +++ | +++ | ++ | |
| | 2H | 95% | | +++ | +++ | +++ | |
| | 4H | 0 | | | | | |

**TABLEAU 9**

| ESSAI 2 | | POURCENTAGE DE RECOUVREMENT | CELLULES DE LA LIGNEE INFLAMMATOIRE | | | DEBRIS CELLULAIRES | FILM PROTEIQUE |
|---|---|---|---|---|---|---|---|
| | | | très faiblement adhérentes | ayant adhéré | ayant excrété | | |
| A | 30 | 90% | + | +++ | + | + | ++ |
| | 1H | 80% | + | +++ | + | + | ++ |
| | 2H | < 5% | | + | + | + | + |
| | 4H | < 5% | | + | + | + | |
| B | 30 | < 5 % | + | + | + | + | |
| | 1 H | < 5% | + | + | + | | |
| | 2 H | < 5 % | + | + | + | + | |
| | 4H | < 5% | | + | + | + | + |
| C | 30 | 15 % | ++ | + | + | + | |
| | 1H | < 5% | | + | + | + | |
| | 2H | < 5% | + | + | + | + | |
| | 4H | 10% | ++ | + | + | + | ++ |
| D | 30 | 100% | ++ | +++ | +++ | +++ | + |
| | 1H | 100% | ++ | +++ | +++ | +++ | + |
| | 2H | -- | -- | -- | -- | -- | |
| | 4H | 100% | ++ | +++ | ++ | ++ | ++ |

**TABLEAU 10**

| ESSAI 3 | | POURCENTAGE DE RECOUVREMENT | CELLULES DE LA LIGNEE INFLAMMATOIRE | | | DEBRIS CELLULAIRES | FILM PROTEIQUE |
|---|---|---|---|---|---|---|---|
| | | | TRES FAIBLEMENT ADHERENTES | ayant adhéré | ayant excrété | | |
| A | 30 | 100% | + | +++ | ++ | ++ | ++ |
| | 1H | 40% | ++ | ++ | ++ | ++ | |
| | 2H | 98% | + | +++ | ++ | ++ | |
| | 4H | 40% | | ++ | +++ | ++ | |
| B | 30 | < 5 % | | + | + | + | + |
| | 1 H | 10% | + | | | + | ++ |
| | 2 H | 100 % | ++ | +++ | + | + | |
| | 4 H | 20% | + | ++ | ++ | ++ | + |
| C | 30 | < 5 % | + | + | + | + | |
| | 1H | 60% | ++ | +++ | ++ | ++ | ++ |
| | 2H | 10% | + | ++ | ++ | ++ | ++ |
| | 4H | 10% | + | ++ | + | + | |
| D | 30 | 95 % | + | +++ | ++ | + | |
| | 1H | 100% | + | +++ | ++ | + | |
| | 2H | 100% | + | +++ | +++ | ++ | |
| | 4H | 100% | ++ | ++ | ++ | ++ | ++ |

## Revendications

1. Dispositif à usage ophtalmologique, notamment implant ou lentille intraoculaire, réalisé en un polymère contenant des atomes d'hydrogène et ayant les qualités optiques et mécaniques traditionnellement requises pour ce type de dispositif, caractérisé en ce que les atomes d'hydrogène présents à la surface dudit polymère ont été remplacés par des atomes de fluor ou des groupements CF, CF₂ ou CF₃, le fluor total représentant au moins 15 % des atomes présents sur cette surface.

2. Dispositif selon la revendication 1, caractérisé en ce que le polymère est du polyméthacrylate de méthyle (PMMA), un polymère de 2-hydroxy-éthyl méthacrylate (HEMA), du silicone ou un polysulfonate.

3. Dispositif selon la revendication 1 ou la revendication 2, caractérisé en ce que le fluor total représente de 30 à 50 % des atomes présents à sa surface.

4. Procédé d'obtention du dispositif selon l'une des revendications 1 à 3 comprenant le traitement de surface dudit dispositif se trouvant dans la forme appropriée pour son utilisation, par un plasma de molécules fluorées non polymérisables, le traitement étant réalisé à une puissance d'émission du réacteur comprise entre environ 3 et environ 10 watts par litre de capacité de reacteur.

5. Procédé selon la revendication 4, caractérisé en ce que les molécules fluorées sont des gaz de tétrafluorure de carbone ou d'hexafluorure de soufre.

6. Procédé selon la revendication 4, caractérisé en ce que le temps de traitement du dispositif est compris entre 1 et 20 minutes.

7. Procédé selon la revendication 4, caractérisé en ce que la température de traitement est comprise entre 20 et 80 °C.

8. Procédé selon la revendication 4, caractérisé en ce que le traitement est effectué sous vide à une pression comprise entre 0,1 et 1 Torr.

9. Procédé selon l'une des revendications 4 à 8, caractérisé en ce que le dispositif est stérilisé après le traitement.

## Claims

1. Device for ophthalmological use, especially an intraocular implant or lens, made of a polymer containing hydrogen atoms and having the optical and mechanical properties traditionally required for this type of device, characterized in that the hydrogen atoms present at the surface of the said polymer have been replaced with fluorine atoms or CF, CF₂ or CF₃ groups, the total fluorine representing at least 15 % of the atoms present on this surface.

2. Device according to Claim 1, characterized in that the polymer is polymethyl methacrylate (PMMA), a 2-hydroxyethyl methacrylate (HEMA) polymer, silicone or a polysulphonate.

3. Device according to Claim 1 or Claim 2, characterized in that the total fluorine represents from 30 to 50 % of the atoms present at its surface.

4. Process for obtaining the device according to one of Claims 1 to 3, including the surface treatment of the said device which is in the appropriate form for its use, with a plasma of nonpolymerizable fluorine-containing molecules, the treatment being carried out at an emission power of the reactor of between approximately 3 and approximately 10 watts per litre of reactor capacity.

5. Process according to Claim 4, characterized in that the fluorine-containing molecules are carbon tetrafluoride or sulphur hexafluoride gases.

6. Process according to Claim 4, characterized in that the period of treatment of the device is between 1 and 20 minutes.

7. Process according to Claim 4, characterized in that the temperature of treatment is between 20 and 80°C.

8. Process according to Claim 4, characterized in that the treatment is performed under a vacuum at a pressure of between 0.1 and 1 torr.

9. Process according to any one of Claims 4 to 8, characterized in that the device is sterilized after treatment.

## Patentansprüche

1. Vorrichtung zur ophthalmologischen Verwendung, insbesondere Implantat oder intraokulare Linse, hergestellt aus einem Polymer, das Wasserstoffatome enthält und die die herkömmlicherweise für solche Vorrichtungen erforderlichen optischen und mechanischen Eigenschaften aufweist, dadurch gekennzeichnet, daß die auf der Oberfläche des Polymers vorhandenenen Wasserstoffatome durch Fluoratome oder durch CF-, CF₂- oder CF₃-Gruppen ersetzt sind, wobei der Gesamtgehalt an Fluor zumindest 15 % der Atome ausmacht, die auf dieser Oberfläche vorhanden sind.

2. Vorrichtung nach Anspruch 1, dadurch gekennzeichnet, daß das Polymer Polymethylmethacrylat (PMMA), ein Polymer aus 2-Hydroxyethylmethacrylat (HEMA), ein Silikon oder ein Polysulfonat ist.

3. Vorrichtung nach Anspruch 1 oder 2, dadurch gekennzeichnet, daß der Gesamtfluorgehalt 30 bis 50 % der auf deren Oberfläche vorhandenen Atome ausmacht.

4. Verfahren zur Herstellung einer Vorrichtung nach einem der Ansprüche 1 bis 3, umfassend die Behandlung der Oberfläche der genannten Vorrichtung, die sich in der zu deren Verwendung geeigneten Form befindet, mit einem Plasma an fluorierten, nicht polymerisierbaren Molekülen, wobei die Behandlung mit einer Ausgangsleistung des Reaktors im Bereich von etwa 3 bis etwa 10 Watt je Liter Reaktorraum durchgeführt wird.

5. Verfahren nach Anspruch 4, dadurch gekennzeichnet, daß die fluorierten Moleküle Gase aus Tetrafluorkohlenstoff oder Schwefelhexafluorid sind.

6. Verfahren nach Anspruch 4, dadurch gekennzeichnet, daß die Behandlungszeit der Vorrichtung im Bereich von 1 bis 20 Minuten liegt.

7. Verfahren nach Anspruch 4, dadurch gekennzeichnet, daß die Behandlungstemperatur im Bereich von 20 bis 80°C liegt.

8. Verfahren nach Anspruch 4, dadurch gekennzeichnet, daß die Behandlung unter Vakuum bei einem Druck im Bereich von 0,1 bis 1 Torr durchgeführt wird.

9. Verfahren nach einem der Ansprüche 4 bis 8, dadurch gekennzeichnet, daß die Vorrichtung nach der Behandlung sterilisiert wird.
